Europäisches Patentamt

European Patent Office

Office européen des brevets

(11) Publication number: **0 363 213**
**A2**

# EUROPEAN PATENT APPLICATION

(21) Application number: **89310243.4**

(22) Date of filing: **06.10.89**

(51) Int. Cl.5 **A61F 2/16**

(30) Priority: **07.10.88 US 255131**

(43) Date of publication of application:
**11.04.90 Bulletin 90/15**

(84) Designated Contracting States:
**AT BE CH DE ES FR GB IT LI NL SE**

(71) Applicant: **IOPTEX RESEARCH INC.**
**1301 Optical Drive**
**Azusa California 91702(US)**

(72) Inventor: **Buboltz, David C.**
**11610 Mount Baldwin Court**
**Rancho Cucamonga California 91701(US)**

(74) Representative: **Cole, William Gwyn**
**Corporate Patents Department Smith and**
**Nephew Research Limited Gilston Park**
**Harlow Essex CM20 2RQ(GB)**

(54) Intraocular lens insertion instrument.

(57) An intraocular lens insertion instrument (10) in-
cluding a holder (12) for holding a folded optic and a
plunger (28) having an enlarged head (32) for forcing
the folded optic from the holder while a haptic of the
lens is protected from damage in a space within the
holder created by a portion of the piston's barrel (34)
having a reduced cross-section.

EP 0 363 213 A2

# INTRAOCULAR LENS INSERTION INSTRUMENT

The present invention relates to deformable intraocular lenses (IOLs) and more particularly to an improved instrument for folding, holding and inserting such an IOL through a small incision in ocular tissue and then releasing the folded IOL for surgical positioning during the implantation of the IOL.

United States Patent 4,573,998; 4,681,102; 4,715,373. 4,747,404; 4,759,359 and 4,473,650 disclose different forms of instruments and methods for folding, inserting and implanting deformable IOLs. Generally, the instruments utilised to hold folded IOLs and to insert the folded IOLs through small incisions in ocular tissue comprise a holder for holding a folded IOL and a plunger or other movable member for ejecting the folded IOL from the holder. With the instruments disclosed in United States Patents 4,681,102 and 4,715,373 for example, the end of a solid cylindrical plunger engages a distal side of a folded optic and the folded superior haptic of the IOL and forces the IOL from a channel or nozzle included in the holder. IOL haptics are extremely thin and fragile. During the ejection process, the forces exerted on the superior haptic often damage the haptic and its connnection to the optic of the IOL. This is particularly common for multi-piece IOLs where the haptics are heat-staked or otherwise separately secured to the lens optic.

The present invention seeks to overcome the disadvantages of the prior art and to provide an IOL ~insertion instrument which does not subject the haptics of a folded IOL to undesired forces or damage during ejection and thereby represent a significant improvement in the art of IOL insertion instruments.

According to the present invention there is provided an intraocular lens (IOL) insertion instrument including an axially open holder for holding an IOL with its optic folded, preferably on its major axis with a haptic, for example its superior haptic extending toward an open distal end of the holder and comprising an axially moveable plunger which includes an enlarged head for insertion into the open distal end of the holder to engage the distal side of the folded optic and a barrel of reduced cross-section extending rearward from the head to define an open space within the holder above the barrel for receiving and containing the first haptic in a relaxed and protected condition as the plunger moves forward in the holder to eject the folded IOL from the open proximal end of the holder.

According to a further embodiment the present invention also provides an instrument for receiving an IOL having two haptics attached thereto or integral therewith which comprises a hand-holdable housing having an IOL holding end which housing has a central bore therethrough, a plunger comprising a barrel having a head attached to or integral with one end thereof, said head having a diameter greater than the diameter of the portion of the barrel to which the head is secured, said plunger being sized to move axially through the central bore within the housing, and means for stopping said plunger's movement through the central bore.

The head of the plunger is preferably of sufficient diameter to contact the outer periphery of the optic portion of an IOL held therein.

The IOL holding end comprises a pair of jaws and the instrument may have means for opening and closing the jaws. Preferably the means for opening and closing the jaws preferably includes means for closing the jaws when an IOL is contained therein so as to fold the IOL about a major axis and to hold it in the folded state without damaging the haptics. Accordingly the jaws may open to an extent sufficient to receive an IOL folded about its major axis. Preferably, therefore the jaws will open sufficiently wide to receive an IOL in a substantially flat configuration.

According to a further embodiment of the present invention, means may be provided for moving the plunger. Such means may include means for moving the plunger axially along the bore through the central housing whereby the head ejects an IOL folded about a major axis.

In a preferred form of the present invention, the instrument comprises a hand-holdable housing having a central bore therethrough, which housing comprises a main portion, a hand grip portion adjacent said main portion and a jaw portion adjacent said hand grip portion, said jaw portion comprising a pair of jaws movable betwen an open position and a closed position, said hand grip portion and said jaw portion having a central bore therethrough actually aligned with the central bore through the main housing portion, a plunger comprising a barrel having a head attached to or integral with one end thereof said head being of greater diameter than the portion of the barrel to which the head is secured, the plunger being axially movable through the central bore in the main housing portion without damaging the haptics, hand grip portion and jaw portion, means for limiting the length of travel of the plunger, means for opening and closing the pair of jaws and means for moving the plunger axially within the central bore within the main hand grip portion and jaw portion.

The invention also includes a method of inserting an IOL folded about a major axis thereof utilizing the instrument in its various embodiments as above described and as well as obvious modifications thereof.

The inferior haptic of the IOL may extend towards an open proximal end of the holder.

The present invention will be further illustrated with reference to the accompanying drawings in which:

Figure 1 is a side view, partially in section, of a preferred form the IOL insertion instrument of the present invention.

Figure 2 is an enlarged top view of the proximal end of the insertion instrument shown in Figure 1 with a deformable IOL held by forceps over open jaws comprising the holder of the instrument.

Figure 3 is a top view of the proximal end of the insertion instrument shown in Figure 2, partially in section and showing the deformable IOL folded and retracted into the holder of the insertion instrument ready for ejection by a movable plunger of the instrument.

Figure 4 is a side view, partially in section, of the proximal end of the insertion instrument shown in Figure 3 and depicted by the dotted circle 4 in Figure 1 and showing the superior haptic of the folded IOL extending into the protective space defined by the reduced cross-section barrel of the plunger.

Figure 5 is an enlarged sectional side view of the portion of the insertion instrument identified by the dotted circle 5 in Figure 1 and showing the manner of connection of a tip carrying the IOL folded jaws to a tubular push rod for axially moving the jaws relative to the housing of the holder.

Figure 6 is an enlarged sectional view of the portion of the insertion instrument identified by the dotted circle 6 in Figure 1 and showing the manner of connection of a control cable to a distal end of the plunger.

Figure 7 is an enlarged view of a gear configuration for reducing the travel of a plunger relative to the travel of a manually actuated drive member to enhance the precision with which the insertion instruments ejected folded IOLs therefrom.

Figure 8 is an enlarged view in section of a further preferred form of an instrument in accordance with the invention.

Referring to the drawings an IOL insertion instrument 10 comprises an axially open holder 12 for holding an IOL 14 with its optic 16 folded preferably on its major axis 18 with its superior haptic 20 extending toward an open distal end 22 of the holder and with its inferior haptic 24 extending toward an open proximal end 26 of the holder. An axially movable plunger 28 supported within a hand-holdable housing 30 includes an enlarged head 32 for insertion into the open distal end 22 of the holder 12 to engage a distal side of the folded optic 16. A barrel 34 of reduced cross-section extends rearward from the head 32 to define an open space 36 within the holder 12 and above the barrel for receiving and containing the superior haptic 20 in a relaxed and protected condition as the plunger 28 moves forward in the holder 12 to eject the folded IOL from the open proximal end 26 of the holder.

The housing 30 comprises an elongated, tubular pencil-like body 38 having an outer tapered proximal end 40 and a counterbore 42 in a distal end thereof defining an annular shoulder 44. The body 38 is adapted to axially receive the holder 12 and the plunger 28 as well as the structure for axially supporting and moving the plunger to eject a folded IOL from the holder.

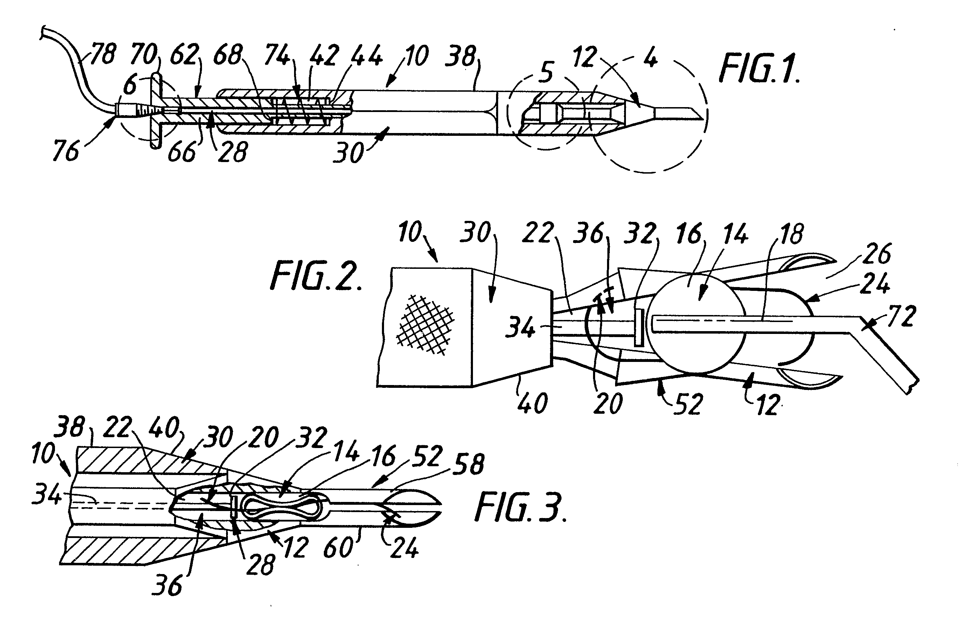

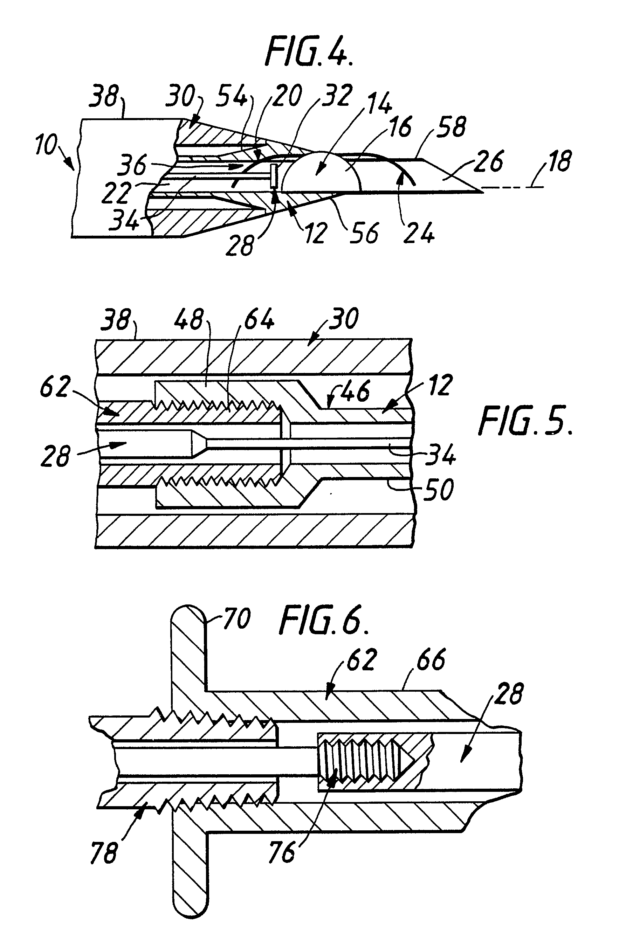

In these regards, the holder 12 preferably comprises an elongated tubular member 46 having a slightly enlarged internally threaded distal end portion 48 shown most clearly in Figure 5, a tubular central portion 50 forward of the distal portion, and a vertically split proximal portion 52 extending forward from the central portion. Between a junction of the proximal portion 52 and the central portion 50 and the opposite end of the proximal portion, the outer surface of the proximal portion tapers outwardly and then inwardly to form two cone-shaped axially extending and opposing collars 54 and 56. Depending forward from the collar 56 are two opposing and axially extending semi-cylindrical jaws 58 and 60 which normally spring outwardly from each other to received and fold the IOL 14 as most clearly shown in Figures 2, 3 and 4. As illustrated in Figure 4 the free ends of the jaws are inclined so that when viewed from the top, the ends of the jaws appear as an ellipse.

The holder 12 is movable axially within the body 38 to close and open the jaws 58 and 60. Such axial movement is under the control of a tubular push rod 62 having an externally threaded forward end mating with the internally threaded portion 48 of the tubular member 46 as shown in Figure 5. A distal end 64 of the push rod 62 is slightly enlarged to slide in and beyond the distal end 48 of the tubular body 46. The end 64 carries an annular flange 70 for hand pushing of the push rod forward in the tubular body 38. As the push rod move forward, the holder 12 moves axially forward out of the housing 30 and the jaws expand to the position shown in Figure 2. In such a position the jaws are ready to receive and folde the IOL 14. As illustrated in Figure 2, the IOL 14 is held by forceps 72 which grip the optic 16 of the IOL preferably along the major axis 18 thereof. Downward movement of the IOL relative to the jaws 58 and 60 will produce a bending of the optic 16 along the major axis 18 as the optic engages semi-cylindrical surfaces of the jaws. Then, as the holder is retracted axially into the body to the position shown in Figures 3 and 4, the jaws close and the optic 16 is folded and moved into the housing 30. Such closing of the jaws and retraction of the holder 12 is automatic upon a release of the flange 70 and is in response to action of a coil spring 74 captured between and bearing continuously upon the shoulders 44 and 68 within the housing. In this regard, the spring 74 continuously urges the push rod 62 rearward. As the push rod moves rearward in response to such spring action, the outer surface of the collar 54 carried by the tubular member 46 engages the proximal end of the tuular body 38 and by cam action forces the jaws towards each other to close the position shown in Figure 3. To open the jaws 58 and 60 to the position shown in Figure 2, simply requires a forward pushing on the flange 70 while holding steady the housing 30.

As described above, once the IOL 14 is folded within the holder 12, it may be ejected from the instrument 10 by operation of the plunger 28. As shown in Figures 2, 3 and 4, the plunger 28 is positioned to extend axially in the tubular body 38 with the enlarged head 32 just to the rear of the jaws 58 and 60. The barrel 34 extends coaxially along and within the tubular member 46 comprising the holder 12 and the tubular rush rod 62 to connect by screw connection to a drive cable 76 as shown most clearly in Figure 6. As there illustrated, the distal end of the barrel 34 is slightly enlarged and internally threaded to received the externally threaded end of the drive cable 76, A sheath 78 for the cable 76 is externally threaded and mates with internal threads at the distal end of the push rod 62 to secure the sheath relative to the cable. Thus arranged, a manual pushing on the drive cable 76 produces a forward movement of the plunger 28 within the housing 30 to move the head 32 through the distal end of the holder and against the distal side of the folded optic 16. Further forward movement of the plunger relative to the holder 12 ejects the folded IOL from the instrument 10 through the open proximal end of the plunger.

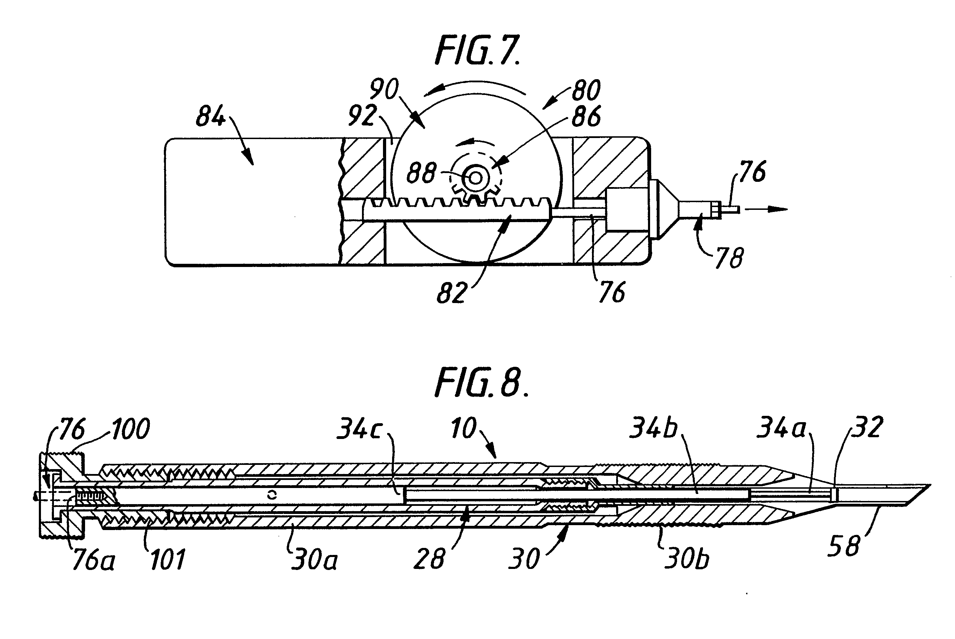

Preferably, the forward and rearward movement of the plunger 28 is under precise control of a gear reduction mechanism 80, such as shown in Figure 7. Generally speaking, the mechanism 80 moves the plunger 28 a relatively short longitudinal distance compared to the travel of a manually actuated drive member for producing the movement of the plunger. This enables an operator to more precisely control movement of the plunger during the IOL ejection operation. In Figure 7, the mechanism 80 is illustrated as comprising an elongated rack 82 connected to the end of the drive cable 76 and supported for longitudinal sliding movement within a housing 84. The housing 84 also supports a pinion gear 86 for turning on a transverse shaft 88 with its teeth engaging the teeth of the rack to drive the rack with a manual turning of the pinion gear. To manually turn the pinion gear 86, thumb wheel 90 is secured thereto and extends through a slot 92 in a side wall of the housing. The thumb wheel 90 is substantially larger in diameter than the pinion gear 86. Hence, the mechanism 80 provides a substantial gear reduction whereby a relatively large turning of the thumb wheel 90 produces a relatively small turning of the pinion gear 86 and a correspondingly small and precisely controllable longitudinal movement of the rack 82, drive cable 76 and plunger 28 during ejection of the folded IOL and subsequent retraction of the plunger.

As previously described, such ejection of the folded IOL is without undesired damage to the haptics of the IOL or their connection to the optic 16. Such damage free ejection of the folded IOL 14 is a by-product of the configuration of the plunger 28 in combination with the holder 12. In particular, as shown in Figures 2, 3 and 4, when the optic 16 is folded by operation of the jaws 58 and 60 as previously described, the superior haptic 20 loops over the head 32 of the plunger 28 and into the open space 36 defined by the reduced cross-section barrel 34 and the tubular member 46 above the barrel. There, the superior haptic contained in a free and protected state while the head of the plunger 28 exerts an ejection force of the folded optic 16. Further, the junction of the haptic and optic is not disturbed or damaged by the plunger as it forces the optic from the holder 12. As previously discussed, such damage-free ejection of deformable IIOL from an insertion instrument represents a substantial improvement over prior instruments.

Not only does the ejection operation of the present instrument represent a substantial improvement over prior instruments, but in addition, the structure and assembly and IOL loading features represent improvements over such instruments. In particular, the structure of the present instrument may be simply and quickly assembled by slipping the spring 74 over the small diameter portion of push rod 62 and then inserting the spring and push rod into the counterbore 42 of the tubular body 38. Next, the threaded end of the tubular member 46 comprising the holder 12 is inserted onto the threaded proximal end of the push rod 62 and while depressing the push rod the holder is rotated to thread the holder onto the push rod. Then the plunger 28 is connected to the drive cable 76 and the plunger fed into the push rod 62. Finally, the sheath 78 for the drive cable 76 is secured to the push rod. Thus assembled, the instrument 10 may be simply and quickly loaded with the IOL 14 by grasping the IOL with the forceps 72 along the major axis 18 of the optic 16 as shown in Figure 2. Holding the instrument 10 in the other hand the push rod 62 is depressed to open the jaws 58 and 60. The IOL is then moved downward into the opening between the jaws and the push road released to produce a closing by action of the spring 74 and a folding of the optic 16. With the IOL folded within the holder 12, the proximal tip of the instrument 10 is inserted into an incision in ocular tissue and the instrument is rotated such that the IOL will unfold posteriorly. While directing the IOL into the posterior capsule, the IOL is ejected by depressing the drive cable 76 to actuate the plunger 28.

Referring to Figure 8, the instrument 10 comprises a housing 30 having a central bore therethrough which housing comprises a main housing portion 30a, a hand grip portion 30b, a jaw portion 58 comprising a pair of jaws which are openable and closeable. The hand grip portion and the jaws have a central bore therethrough axially aligned with the central bore through the main housing portion 30a. Plunger 28 comprises a first barrel portion 34a of a diameter less than that of head 32 which is attached to or integral with one end thereof, second barrel portion 34b which is of a diameter substantially equal to the diameter of head 32 and a third barrel portion 34c of a diameter greater than that of the second barrel portion. In the end of third barrel portion 34c there is a recess suitable for receiving a cable 76a which is secured to cable drive 76 for moving the barrel axially along the central bore whereby the head 32 moves into contact with the optic of the folded IOL to eject the folded IOL. Suitable means for opening and closing the jaws may comprise means 100, screw threaded at 101 to the interior of the main housing portion 30a.

## Claims

1. An intraocular lens insertion instrument, comprising:
an axially open holder for holding an intraocular lens with its optic folded and a haptic extending toward an open distal end of the holder; and
an axially movable plunger having
an enlarged head for insertion into the open distal end of the holder to engage a distal side of the folded optic, and
a barrel of reduced cross-section extending rearward from the head to define an open space within the holder above the barrel for receiving and containing the haptic in a relaxed and protected condition as the plunger moves forward in the holder to eject the folded IOL from an open proximal end of the holder.

2. An instrument according to claim 1 comprising means for supporting and mechanically moving the plunger in forward and rearward directions between the open distal and proximal ends of the holder.

3. An instrument according to claim 1 or claim 2 wherein the holder comprises first and second forwardly extending jaws for opening to received the intraocular lens with its optic folded and its haptic extending rearward toward the distal open end of the holder, and for closing to hold the intraocular lens in its folded condition.

4. An instrument according to claim 3 comprising:
means for mechanically opening and closing the jaws; and
means for supporting and mechanically moving the plunger forward and rearward in a plane lying between the jaws to eject the folded intraocular lens from the jaws through the open proximal end of the holder with forward movement of the plunger.

5. An instrument according to claim 3 or 4 further comprising:
a tubular hand-holdable housing;
a tubular push rod axially movable within the housing with a rear end portion extending beyond an open rear end of the housing;
means securing the jaws to a forward end portion of the push rod to cantilever in an open condition through and outward beyond a forward open end of the housing;
spring means within the housing for urging the push rod in a rearward direction relative to the housing to carry the jaws into and against the forward end of the housing to move to a closed condition; and
manually actuated mechanical means for moving the plunger within the push rod toward and between the jaws to eject the folded IOL therefrom.

6. An instrument according to any one of the preceding claims wherein the means for mechanically moving the plunger includes
a manually movable drive member, and
gear means between the drive member and the plunger for moving the plunger a relatively short distance compared to travel of the drive member whereby movement of the plunger may be precisely controlled.

7. An instrument for receiving an intraocular lens having at least one haptic attached thereto or integral therewith which comprises a housing having a lens holding end which housing has a central bore therethrough, a plunger comprising a barrel having a head attached to or integral with one end thereof, said head having a diameter greater than the diameter of the portion of the barrel to which the head is secured, said plunger being sized to move axially through the central bore within the housing, and means for stopping said plunger's movement through the central bore.

8. An instrument according to claim 7 wherein the lens holding end comprises a pair of jaws and wherein the instrument further comprises means for opening and closing the jaws.

9. An instrument according to claim 7 or 8 which further comprises means for moving the plunger axially along the central bore within the housing.

10. An instrument according to any of claims 7 to 9 wherein the head is of sufficient diameter to contact the outer periphery of the optic portion of an intraocular lens held therein.

11. An instrument according to any one of the claims 8 to 10 wherein the jaws open sufficiently wide to receive an IOL in a substantially flat configuration.

12. An instrument according to claims 8 to 11 wherein the means for opening and closing the jaws include means for closing the jaws when an IOL is contained therein so as to fold the IOL about a major axis.

13. An instrument according to any one of claims 7 to 12 wherein the means for moving the plunger includes means for moving the plunger axially along the bore through the central housing whereby the head ejects an intraocular lens folded about a major axis.

FIG.1.

FIG.2.

FIG.3.

EP 0 363 213 A2

# FIG.4.

# FIG.5.

# FIG.6.

EP 0 363 213 A2

## FIG.7.

## FIG.8.